# EUROPEAN PATENT APPLICATION

(11) **EP 2 039 288 A1**
(43) Date of publication of application: **25.03.2009**
(21) Application number: 08016141.7
(22) Date of filing: 12.09.2008
(51) Int. Cl.: A61B 5/00, G01N 21/64

(54) **Dental observation apparatus**

(30) Priority: 18.09.2007 JP 2007241363; 11.07.2008 JP 2008181574
(71) Applicant: Olympus Corporation, Tokyo 151-0072 (JP)
(72) Inventor: Kaneko, Mamoru, Hannou-shi, Saitama 357-0041 (JP); Kumada, Yoshiyuki, Komae-shi, Tokyo 201-0011 (JP); Yoshida, Kazuhiro, Sagamihara-shi, Kanagawa 229-1134 (JP)
(74) Representative: von Hellfeld, Axel

(57) **Abstract**

Caries located at a position that cannot be directly viewed, such as one of the adjacent surfaces between teeth, can be observed with high contrast, and the spread and the degree of invasion of the caries can be observed. A dental observation apparatus has: an irradiating unit radiating illumination light including an infrared region; a detecting unit separately detecting fluorescence generated from a caries portion by irradiation with the illumination light and scattered light of the illumination light at the tooth; and an image processing unit which forms a fluorescence image based on the fluorescence detected by the detecting unit, which forms a scattered light image capable of identifying a boundary between an enamel layer and a dentine layer, the layers having different scattering properties, based on the intensity of the scattered light detected by the detecting unit, and which combines the fluorescence image and the scattered light image.

## Description

### BACKGROUND OF THE INVENTION

### 1. FIELD OF THE INVENTION

The present invention relates to a dental observation apparatus.

This application is based on Japanese Patent Applications, No. 2007-241363 and No. 2008-181574, the content of which is incorporated herein by reference.

### 2. DESCRIPTION OF RELATED ART

Heretofore, dental caries is generally recognized by visual inspection or x-ray inspection by a dentist. In visual inspection, it is inconveniently difficult to confirm very small caries at an early stage and/or caries located at a position that cannot be directly viewed, such as one of the adjacent surfaces between teeth. In addition, one problem with x-ray inspection is that inspection cannot be frequently performed because x-ray exposure occurs.

In order to avoid the above inconveniences to detect dental caries at an early stage and to observe caries located at a position which cannot be easily viewed, there is a known technique in which teeth are irradiated with external white light, and light passing through the teeth forms an image (for example, see US Patent No. 6,201,880).

However, the technique disclosed in US Patent No. 6,201,880 has drawback that, since white light which is liable to be influenced by scattering inside teeth is used, caries occurring at a position that cannot be directly viewed from the surface of teeth cannot be observed with high contrast.

### BRIEF SUMMARY OF THE INVENTION

The present invention has been conceived in consideration of the above-described situation, and an object of the present invention is to provide a dental observation apparatus that can observe with high contrast caries located at a position that cannot be directly viewed, such as one of the adjacent surfaces between teeth, and also that can observe the spread and the degree of invasion of the caries.

In order to achieve the above object, the present invention provides the following solutions.

The present invention provides a dental observation apparatus comprising: an irradiating unit radiating illumination light including an infrared region; a detecting unit separately detecting fluorescence generated from a caries portion of a tooth by radiation of the illumination light and scattered light of the illumination light at the tooth; and an image processing unit which forms a fluorescence image based on the fluorescence detected by the detecting unit, which forms a scattered light image capable of identifying a boundary between an enamel layer and a dentine layer, the layers having different scattering properties, based on the intensity of the scattered light detected by the detecting unit, and which combines the fluorescence image and the scattered light image.

According to the present invention, when the illumination light including an infrared region is radiated from the irradiating unit to the tooth, the fluorescence is emitted from the caries portion present in the tooth, and in addition, the scattered light of the illumination light is generated from the whole tooth; hence, when the above two types of light are detected separately by the detecting unit, and the fluorescence image and the scattered light image are combined, a composite image including the whole tooth and the caries portion located therein can be obtained with high contrast.

When the fluorescence and the scattered light are detected in a time-division manner or are detected at different wavelengths by selection of a fluorescent dye, the two types of light can be separately detected. Hence, the caries portion located at a position that cannot be directly viewed, such as one of the adjacent surfaces between teeth, can be clearly detected.

In this case, since the scattering properties of the enamel layer and the dentine layer of the tooth are different from each other, the intensity of the scattered light from the enamel layer and that from the dentine layer are different from each other. Hence, in the image processing unit, the scattered light image capable of identifying the boundary between the enamel layer and the dentine layer can be obtained based on the difference in intensity of the scattered light. As a result, the position of the caries portion with respect to the boundary can be confirmed; hence, the spread and the degree of invasion of the caries portion can be easily observed.

In the present invention, the image processing unit may compare the intensity of the scattered light obtained by the detecting unit with a predetermined threshold value and form the scattered light image capable of identifying the boundary between the enamel layer and the dentine layer.

As described above, since the scattering properties of the enamel layer and the dentine layer of the tooth are different from each other, the intensity of the scattered light from the enamel layer and that from the dentine layer are different from each other. Hence, when an appropriate threshold value is determined beforehand, the two types of scattered light can be discriminated, and hence a scattered light image capable of identifying the boundary can be easily formed.

In addition, in the present invention, the irradiating unit may further radiate visible light, the detecting unit may further detect scattered light of the visible light, and the image processing unit may form a scattered visible light image based on the intensity of the visible light detected by the detecting unit, so that by comparison with the scattered visible light image, the scattered light image capable of identifying the boundary between the enamel layer and the dentine layer is generated.

With the structure described above, the visible light radiated from the irradiating unit is detected by the detecting unit, and the scattered visible light image formed based on the intensity of the visible light thus detected and the scattered light image obtained by radiation of the illumination light including an infrared region are compared with each other by the image processing unit. Accordingly, the scattered light from the enamel layer and that from the dentine layer of the tooth can be clearly discriminated from each other. For the comparison, for example, difference calculation or division may be performed between the images.

In addition, in the present invention, the image processing unit may perform the combining by imparting different colors to regions corresponding to the caries portion in the fluorescence image, and the enamel layer and the dentine layer in the scattered light image.

Accordingly, the caries portion, the enamel layer, and the dentine layer can be clearly and distinctly displayed, and the spread and the degree of invasion of the caries portion with respect to the boundary between the enamel layer and the dentine layer can be easily observed.

In addition, in the present invention, the dental observation apparatus may further comprise: a determination unit determining the degree of invasion of the caries portion by comparing the distance from the surface of the enamel layer to the boundary with the distance from the caries portion to the boundary.

With the structure described above, when the distance from the caries portion to the boundary with respect to the distance from the surface of the enamel layer to the boundary is smaller than a predetermined ratio, the determination unit can determine that the degree of invasion of the caries portion is high. Hence, in tooth treatment, an appropriate treatment can be easily selected.

In addition, in the present invention, the irradiating unit may include a first irradiating unit radiating first illumination light from a side surface of the tooth and a second irradiating unit radiating second illumination light from an occluding surface of the tooth, and the detecting unit may be disposed to face the first irradiating unit with the tooth interposed therebetween.

With the structure described above, when the first illumination light radiated from the side surface of the tooth and passing therethrough is detected by the detecting unit, based on the difference in intensity of scattered light at the tooth, a scattered light image capable of identifying the boundary between the enamel layer and the dentine layer can be obtained. In addition, the second illumination light radiated from the occluding surface of the tooth excites a fluorescent substance accumulated at the caries portion present in the tooth, so that fluorescence is emitted. In this case, since the fluorescence generated by the second illumination light incident from the occluding surface is detected by the detecting unit disposed to face the side surface of the tooth, the incident second illumination light is not likely to be detected by the detecting unit, and hence a fluorescence image having a high contrast can be obtained. As a result, even a caries portion located at a position that cannot be directly viewed, such as one of the adjacent surfaces between teeth, can be clearly detected.

In addition, in the above structure, the dental observation apparatus may further comprise: a first polarizing member disposed between the first irradiating unit and the tooth, and a second polarizing member which is disposed between the tooth and the detecting unit and which has a polarization direction different from that of the first polarizing member.

For example, the first illumination light passing between teeth and reflection light of the first illumination light at the teeth are prevented from being directly incident on the detecting unit by the first and the second polarizing members, and hence a scattered light image having a high contrast can be obtained.

In addition, in the structure described above, the second illumination light is preferably near infrared light.

When near infrared light is used, the autofluorescence is suppressed, and a fluorescence image having a high contrast can be obtained from agent fluorescence.

In addition, in the structure described above, the dental observation apparatus may further comprise: a blocking unit which is disposed between the tooth and the detecting unit and which transmits the first illumination light and blocks the second illumination light.

According to the structure described above, when the first illumination light is radiated, the first illumination light scattered by the tooth passes through the blocking unit and is detected by the detecting unit, so that the scattered light image can be obtained. On the other hand, when the second illumination light is radiated, for example, the second illumination light reflected at the tooth is blocked by the blocking unit and is prevented from entering the detecting unit. Accordingly, a fluorescence image having a high contrast can be obtained.

In addition, in the above structure, the dental observation apparatus may further comprise an illumination light switching unit switching between the first illumination light and the second illumination light in a time-division manner, and the detecting unit may detect the fluorescence or the scattered light in synchronization with switching timing of the illumination light by the illumination light switching unit.

Accordingly, since the fluorescence or the scattered light is detected by the detecting unit when the first illumination light and the second illumination light are respectively radiated, which are switched in a time-division manner by the illumination light switching unit, the fluorescence image and the scattered light image can be obtained by one single detecting unit without causing any influence on each other.

In addition, in the present invention, the dental observation apparatus may further comprise: an insertion unit which can be inserted into an oral cavity, and the irradiating unit may be a semiconductor light source disposed in a front end part of the insertion unit.

Accordingly, the dental observation apparatus can be compactly designed.

In addition, in the above structure, the first illumination light and the second illumination light may be near infrared light; an illumination light switching unit switching between the first illumination light and the second illumination light in a time-division manner and a blocking unit blocking the second illumination light from entering the detecting unit when the second illumination light is switched on by the illumination light switching unit may be provided; and the detecting unit may detect the fluorescence or the scattered light in synchronization with switching timing of the illumination light by the illumination light switching unit.

Accordingly, even when near infrared rays having equivalent wavelength bands are used as the first and the second illumination light, the fluorescence image and the scattered light image can be obtained by one single detecting unit without causing any influence on each other. A filter that can be inserted in and removed from a light path or a switchable liquid crystal filter may be used as the blocking unit.

Accordingly, the present invention affords advantages in that caries located at a position that cannot be directly viewed, such as one of the adjacent surfaces between teeth, can be observed with high contrast, and in that the spread and the degree of invasion of the caries can be observed.

### BRIEF DESCRIPTION OF THE SEVERAL VIEWS OF THE DRAWINGS

Fig. 1 is an overall structural view showing a dental observation apparatus according to a first embodiment of the present invention.
Fig. 2 is a graph showing the transmittance of each filter in the dental observation apparatus shown in Fig. 1.
Fig. 3 is a view showing one example of a fluorescence image obtained by the dental observation apparatus shown in Fig. 1.
Fig. 4 is a graph showing general scattering properties of a tooth.
Fig. 5 is a view showing one example of a scattered light image obtained by the dental observation apparatus shown in Fig. 1.
Fig. 6 is a view showing one example of a scattered light image obtained by processing the scattered light image shown in Fig. 5 so as to clearly show the boundary between an enamel layer and a dentine layer.
Fig. 7 is a view showing one example of a composite image obtained by combining the fluorescence image shown in Fig. 3 and the scattered light image shown in Fig. 6.
Fig. 8 is an overall structural view showing a first modification of the dental observation apparatus shown in Fig. 1.
Fig. 9 is an overall structural view showing a second modification of the dental observation apparatus shown in Fig. 1.
Fig. 10 is an overall structural view showing a third modification of the dental observation apparatus shown in Fig. 1.
Fig. 11 is an overall structural view showing a fourth modification of the dental observation apparatus shown in Fig. 1.
Fig. 12 is an overall structural view showing a fifth modification of the dental observation apparatus shown in Fig. 1.
Fig. 13 is a graph showing the transmittance of each filter in the dental observation apparatus shown in Fig. 12.
Fig. 14 is an overall structural view showing a sixth modification of the dental observation apparatus shown in Fig. 1.
Fig. 15 is a graph showing the transmittance of each filter in the dental observation apparatus shown in Fig. 14.
Fig. 16 is an overall structural view showing a seventh modification of the dental observation apparatus shown in Fig. 1.
Fig. 17 is an overall structural view showing an eighth modification of the dental observation apparatus shown in Fig. 1.
Fig. 18 is a graph showing the transmittance of each filter of the dental observation apparatus shown in Fig. 17.
Fig. 19 is an overall structural view showing a ninth modification of the dental observation apparatus shown in Fig. 1;
Fig. 20 is a view showing one example of determination of the degree of invasion of a caries portion by the dental observation apparatus shown in Fig. 1.
Fig. 21 is an overall structural view showing a dental observation apparatus according to a second embodiment of the present invention.
Fig. 22 is a side view showing the arrangement of a first and a second irradiating unit of the dental observation apparatus shown in Fig. 21.
Fig. 23 is a view showing wavelength regions of first and second illumination light and the transmittance of each light-receiving filter in the dental observation apparatus shown in Fig. 21.
Fig. 24 is a timing chart showing switching timing of the first and the second illumination light of the dental observation apparatus shown in Fig. 21.
Fig. 25 is a graph showing the relationship between the wavelength of illumination light and transmission characteristic.
Fig. 26 is a graph showing the relationship between the wavelength of illumination light and the intensity of autofluorescence.
Fig. 27A is a photograph showing an agent fluorescence image of a caries portion at a front surface of a sliced molar.
Fig. 27B is a photograph showing an agent fluorescence image of a caries portion at a rear surface of the sliced molar.
Fig. 28 is a photograph showing an agent fluorescence image obtained when the second illumination light is incident from an occluding surface.
Fig. 29 is a perspective view illustrating an arrangement example of optical fibers of an insertion unit to be inserted into an oral cavity.
Fig. 30 is a perspective view illustrating an arrangement example of a semiconductor light source of the insertion unit to be inserted into an oral cavity.
Fig. 31 is a view showing a modification of the wavelength regions of the first and second illumination light and the transmittance of each light-receiving filter in the dental observation apparatus shown in Fig. 21.
Fig. 32 is a view showing another modification of the wavelength regions of the first and second illumination light and the transmittance of each light-receiving filter of the dental observation apparatus shown in Fig. 21.

### DETAILED DESCRIPTION OF THE INVENTION

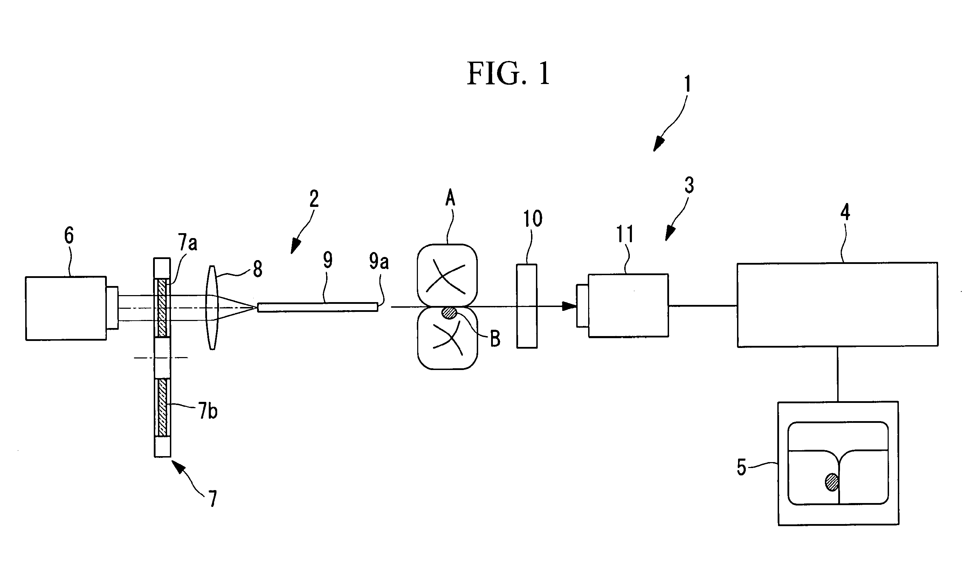

A dental observation apparatus 1 according to a first embodiment of the present invention will be described with reference to Figs. 1 to 7.

The dental observation apparatus 1 according to this embodiment has, as shown in Fig. 1, an irradiating unit 2 irradiating a tooth A with illumination light including an infrared region, a detecting unit 3 detecting light emitted from a caries portion B of the tooth A by irradiation with the illumination light from the irradiating unit 2, an image processing unit 4 forming an image based on the intensity of light detected by the detecting unit 3, and a display unit 5 displaying the image formed by the image processing unit 4.

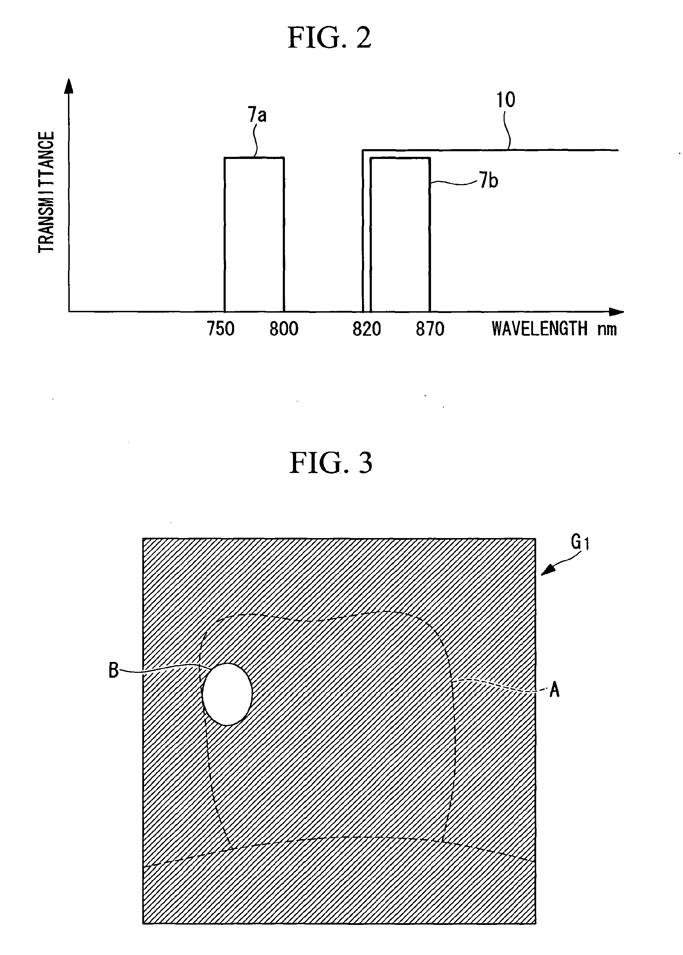

The irradiating unit 2 includes a light source 6 generating light in a wide wavelength band, a rotary filter 7 which includes two types of filters 7a and 7b selecting two wavelength bands (such as 750 to 800 nm and 820 to 870 nm) in different infrared regions from light emitted from the light source 6, as shown in Fig. 2, a focusing lens 8 focusing the illumination light selected by the rotary filter 7, and an optical fiber 9 which guides the illumination light focused by the focusing lens 8 and then radiates the light onto the tooth A from an emission end of the fiber 9.

The light source 6 may be, for example, one selected from a light emitting diode (LED), a super luminescent diode (SLD), a halogen lamp, a xenon lamp, and a laser light source.

The detecting unit 3 is disposed opposite to the irradiating unit 2 with the tooth A interposed therebetween and has a light-receiving filter (blocking unit) 10 to block light in a predetermined wavelength band (such as 820 nm or less) incident from the tooth A and a light detector 11 detecting light passing through the light-receiving filter 10. The light detector 11 is, for example, an image acquisition element, such as CCD or CMOS detector.

The image processing unit 4 is designed so as to form a fluorescence image based on the intensity of fluorescence detected by the detecting unit 3 when the illumination light in a first wavelength band (750 to 800 nm) is radiated to the tooth A and so as to form a scattered light image based on the intensity of scattered light detected by the detecting unit 3 when the illumination light in a second wavelength band (820 to 870 nm) is radiated to the tooth A. That is, the image processing unit 4 is designed to generate the fluorescence image and the scattered light image in synchronization with the rotation of the rotary filter 7.

In addition, the image processing unit 4 is designed to generate a scattered light image by binarizing the scattered light image thus generated based on a predetermined threshold value.

Furthermore, the image processing unit 4 is designed to output an image by combining the fluorescence image and the scattered light image thus generated on the display unit 5.

The operation of the dental observation apparatus 1 according to this embodiment will now be described.

When the tooth A is observed by the dental observation apparatus 1 according to this embodiment, after an appropriate fluorescent agent (such as indocyanine green (ICG)) is applied to the tooth A, an emission end 9a of the optical fiber 9 is disposed to face the tooth A, the detecting unit 3 is disposed opposite to the optical fiber 9 with the tooth A interposed therebetween, and light is then emitted from the light source 6.

Subsequently, when one filter 7a is disposed in a light path by rotating the rotary filter 7, the illumination light in the first wavelength band (750 to 800 nm) is focused by the focusing lens 8, is then guided by the optical fiber 9, and is finally radiated to the surface of the tooth A. When the illumination light in this wavelength band is radiated, the fluorescent agent accumulated at the caries portion B is excited, so that fluorescence is generated.

The fluorescence thus generated is emitted in all directions, is then made to pass through the light-receiving filter 10 disposed to face the tooth A, and is finally detected by the light detector 11. An intensity signal of two-dimensional fluorescence detected by the light detector 11 is sent to the image processing unit 4, so that a fluorescence image G1 is generated. Since the light-receiving filter 10 is designed so as to block the illumination light in the first wavelength band, the illumination light itself is not allowed to pass through the light-receiving filter 10, and only the fluorescence is detected by the light detector 11. That is, at this stage, as shown in Fig. 3, the fluorescence image G1 having luminance only at the caries portion B is generated.

On the other hand, when the other filter 7b of the rotary filter 7 is disposed in the light path, the illumination light in the second wavelength band (820 to 870 nm) is focused by the focusing lens 8, is then guided by the optical fiber 9, and is finally radiated onto the surface of the tooth A. Unlike the illumination light in the first wavelength band, this illumination light in the second wavelength band does not excite the fluorescent agent, and hence no fluorescence is emitted. However, the illumination light is scattered at places inside the tooth A and is then emitted from the tooth A in the form of scattered light.

Since the scattered light thus emitted includes a wavelength band that can pass through the light-receiving filter 10 disposed to face the tooth A, the scattered light passes through the light-receiving filter 10 and is then detected by the light detector 11.

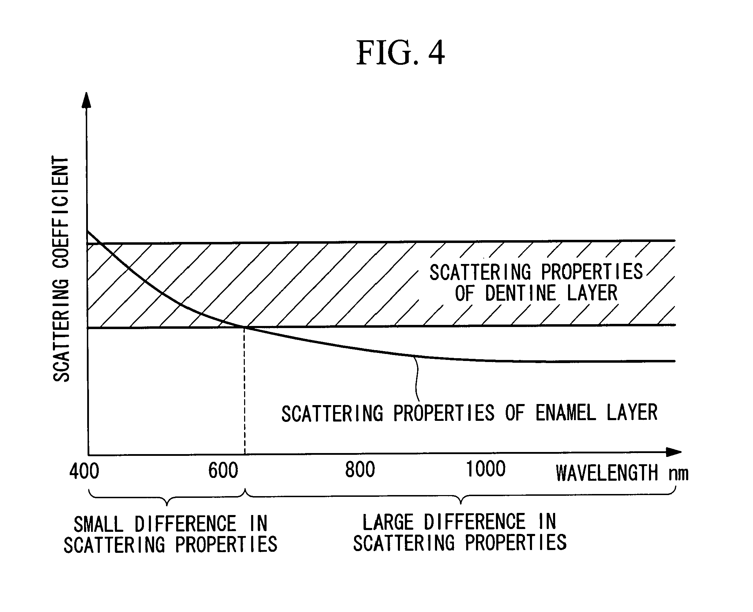

In this case, the tooth A is generally formed of an enamel layer A₁ located at the surface side and a dentine layer A₂ located at the inner side, and scattering properties of the enamel layer A₁ and those of the dentine layer A₂ are different from each other, as shown in Fig. 4. This difference in scattering properties becomes particularly significant in an infrared region.

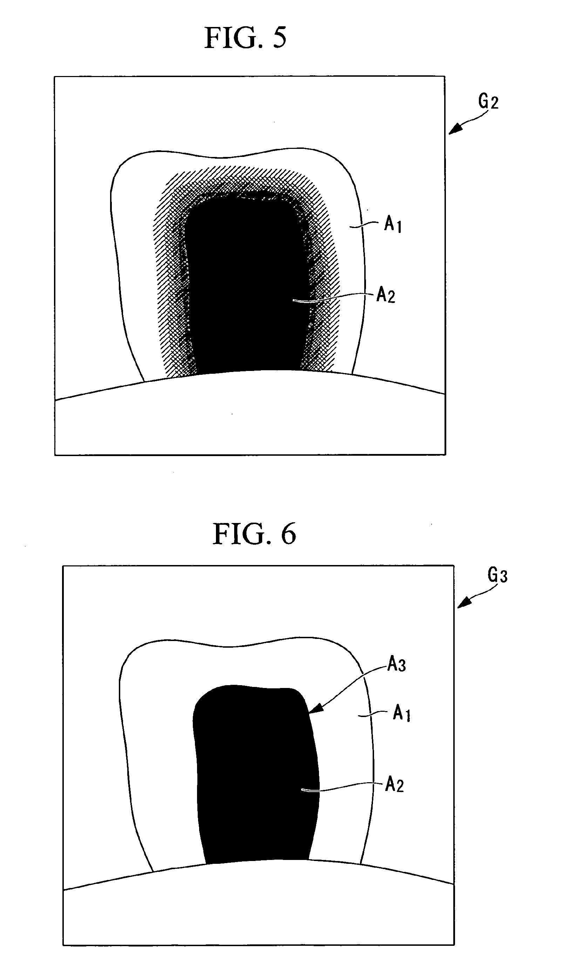

Hence, the intensity of scattered light received by the detecting unit 3 through the enamel layer A₁ is different from the intensity of scattered light received by the detecting unit 3 through the dentine layer A₂, and as shown in Fig. 5, a scattered light image G₂ in which the dentine layer A₂ is dark and the enamel layer A₁ is bright is obtained.

In the image processing unit 4, when the obtained scattered light image G₂ is binarized with respect to a predetermined threshold, a scattered light image G₃ can be generated in which a boundary A₃ between the enamel layer A₁ and the dentine layer A₂ is clearly shown, as shown in Fig. 6.

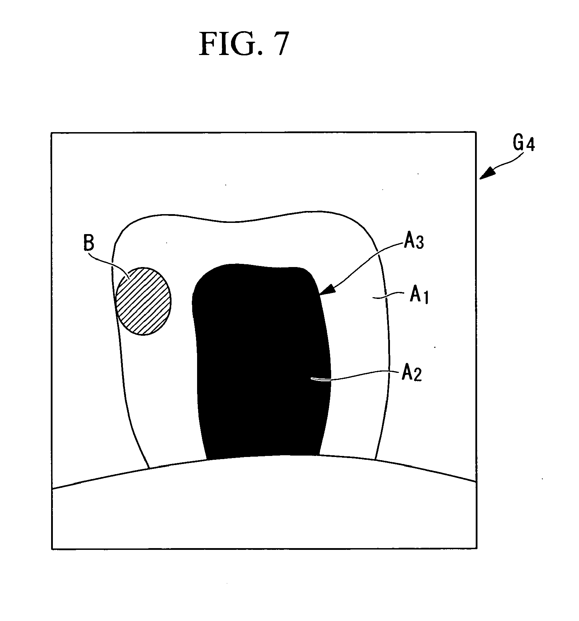

Furthermore, when the fluorescence image G₁ and the scattered light G₃ thus generated are combined by the image processing unit 4, a composite image G₄ clearly showing the caries portion B can be generated in which the boundary A₃ between the enamel layer A₁ and the dentine layer A₂ is clearly shown in the overall image, as shown in Fig. 7.

As described above, according to the dental observation apparatus 1 according to this embodiment, since the fluorescent agent accumulated at the caries portion B located at a position that cannot be directly viewed, such a surface of the tooth A facing an adjacent tooth, is excited by illumination light in an infrared region, which is not likely to be scattered, and the fluorescence image G₁ including this caries fluorescence image with a high contrast is combined with the scattered light image G₃ of the overall tooth A in which the boundary A₃ between the enamel layer A₁ and the dentine layer A₂ is clearly shown by using the difference in scattering properties, this embodiment affords an advantage in that the spread and the degree of invasion of the caries portion B of the tooth A can be more clearly observed.

In this embodiment, although the wavelength band of the illumination light to be radiated is described by way of example, it is not limited thereto, and arbitrary illumination light including an infrared region may also be used. In addition, the fluorescent agent may be arbitrarily selected. For example, instead of the light in only an infrared region, illumination light in both a visible light region and an infrared region may also be used. In this case, the light in a visible light region is used to excite a fluorescent agent accumulated at the caries portion B for emission of fluorescence. On the other hand, the light in an infrared region is scattered at places inside the tooth A and is then detected by the light detector 11 as scattered light. In this case, an agent that is excited by light in a visible light region to emit fluorescent light may be used as the fluorescent agent. In addition, the rotary filter 7 may be designed to have two types of filters that select illumination light in a visible light region and illumination light in an infrared region.

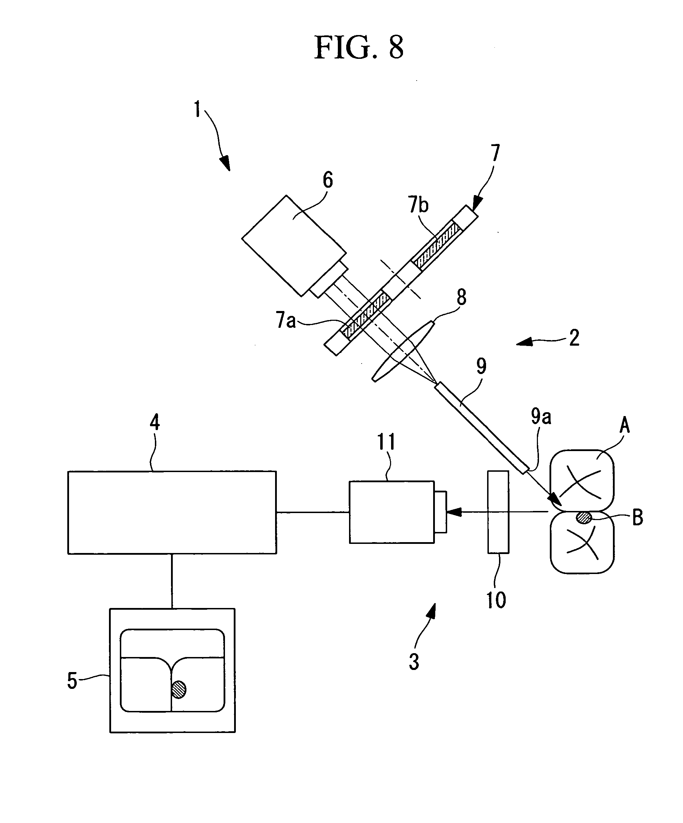

In addition, the method used in this embodiment involves disposing the irradiating unit 2 and the detecting unit 3 to face each other with the tooth A interposed therebetween so that the scattered light passing through the tooth A is detected; however, instead of the above method, as shown in Fig. 8, it is also possible to use a method in which scattered light that is reflected to the same side as that at which illumination light is radiated onto the tooth A is detected.

In this case, in the scattered light image G₂ obtained by the light detector 11, the above luminance distribution is reversed. In addition, although the illumination light is radiated from one side of the detecting unit 3 provided in front of the tooth A, as shown in Fig. 8, instead of this structure, illumination light may be radiated from both sides of the detecting unit 3.

In addition, in this embodiment, the scattered light image G₂ is binarized using a predetermined threshold to generate the scattered light image G₃ in which the boundary A₃ between the enamel layer A₁ and the dentine layer A₂ is clearly shown; however, instead of the above, the difference between the scattered image G2 and a scattered visible light image (not shown) obtained by radiation of visible light or the ratio therebetween may be computed so as to clearly show the boundary A₃ between the enamel layer A₁ and the dentine layer A₂. In this case, in order to radiate visible light, the rotary filter 7 may have a third filter through which visible light passes.

Subsequently, after the levels of the two scattered light images thus obtained are made to coincide with each other, difference computation of the two images is performed so that the difference in intensity distribution based on the difference in scattering properties of the enamel layer A₁ and the dentine layer A₂ is enhanced; hence, an overall image of the tooth A in which the boundary A₃ is more precisely and clearly shown can be generated.

In addition, different colors may be imparted to the individual regions of the caries portion B of the fluorescence image G₁ and the enamel layer A₁ and the dentine layer A₂ of the scattered light image G₃ thus obtained so as to display the above regions in different colors.

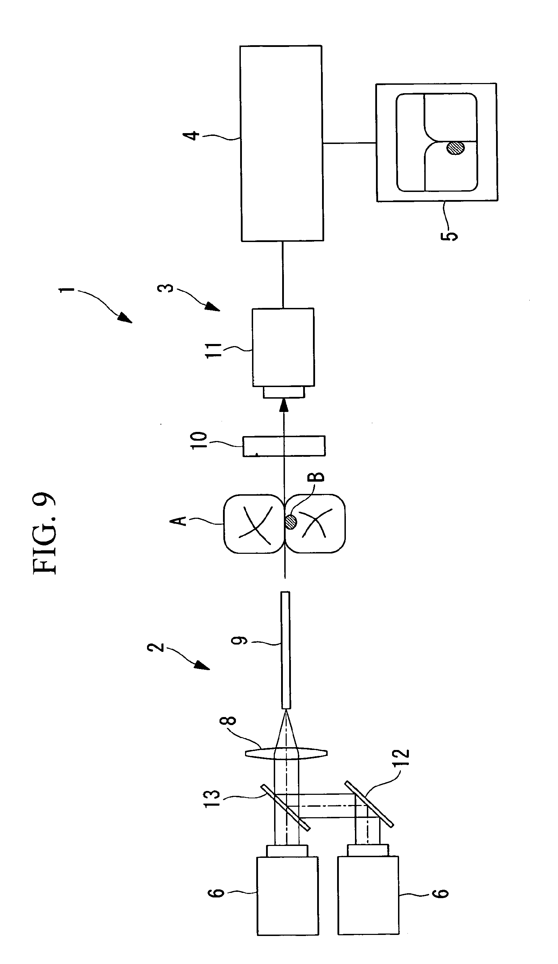

In this embodiment, the two types of illumination light are generated by the rotary filter 7; however, as shown in Fig. 9, at least two light sources 6 may be provided so that illumination light in different wavelength bands emitted therefrom is made to enter the same light path by a mirror 12 and a dichroic mirror 13.

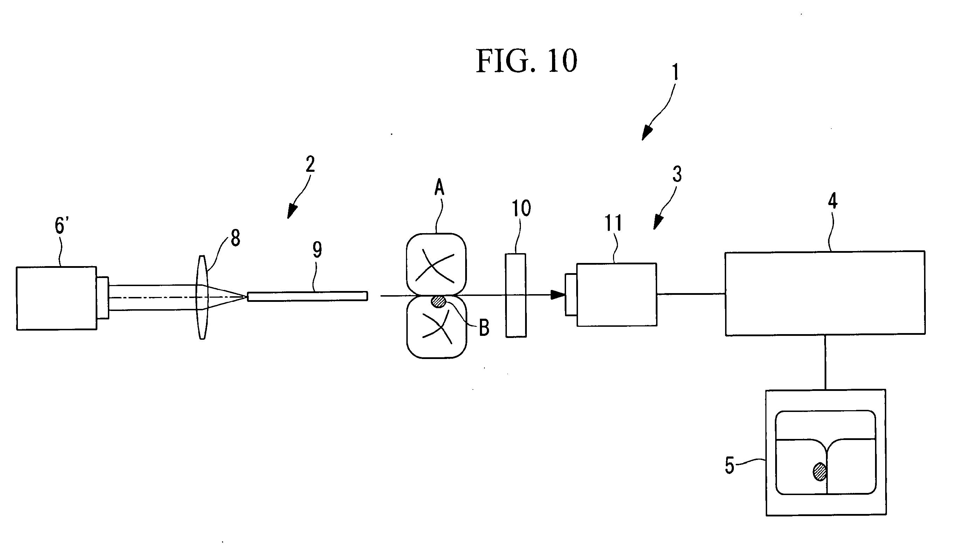

In addition, as shown in Fig. 10, without using the rotary filter 7, two types of illumination light may be generated by a wavelength-tunable laser light source 6'.

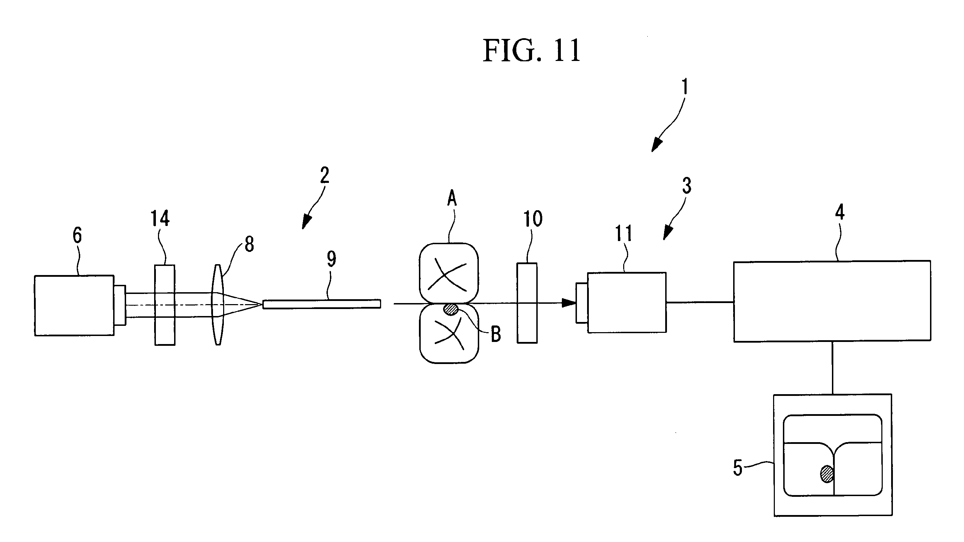

In addition, as shown in Fig. 11, instead of the rotary filter 7, a liquid crystal filter 14 or a comb filter may also be used.

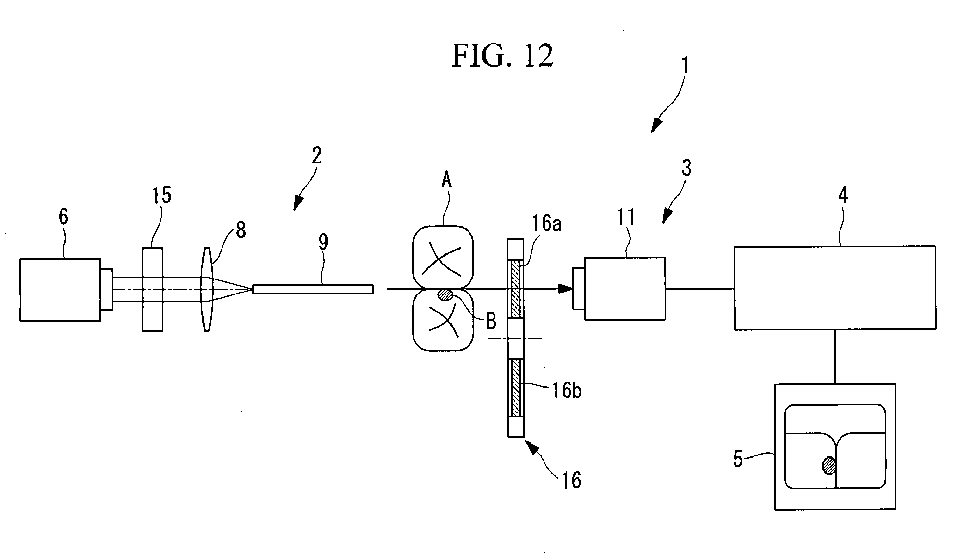

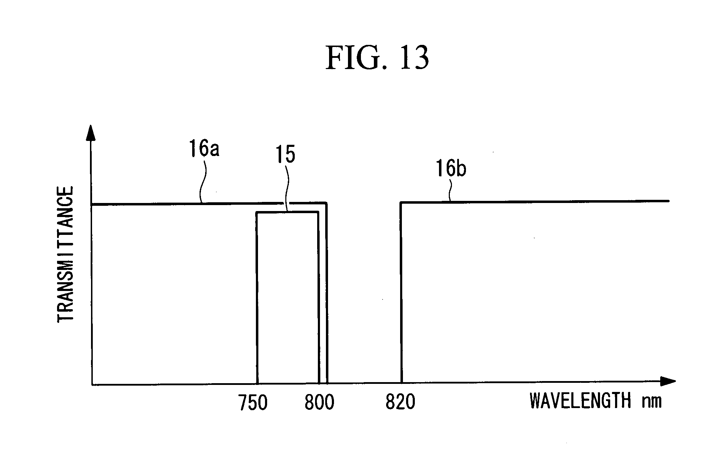

In addition, as shown in Fig. 12, a fixed irradiation filter 15 may be provided instead of the rotary filter 7, and instead of the light-receiving filter 10, a rotary filter 16 may be provided. In this case, for example, as shown in Fig. 13, the irradiation filter 15 may be designed to transmit light in a wavelength band of 750 to 800 nm, and the rotary filter 16 at the detecting unit 3 side may have a filter 16a for scattered light observation which transmits light in a wavelength band of 800 nm or less and a filter 16b for fluorescence observation which transmits light in a wavelength band of 820 nm or more.

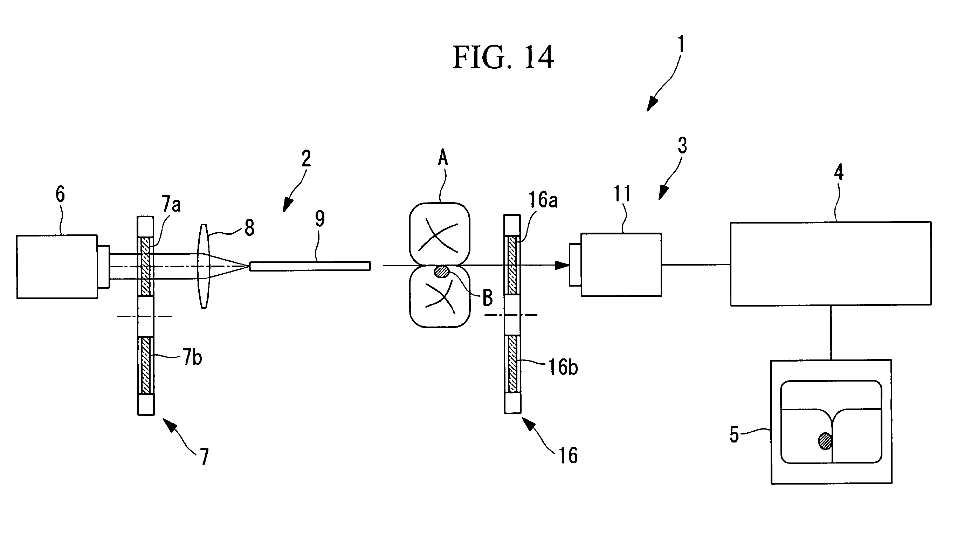

In addition, as shown in Fig. 14, the rotary filters 7 and 16, which are to be synchronously rotated, may be provided at the irradiating unit 2 and the detecting unit 3 sides, respectively.

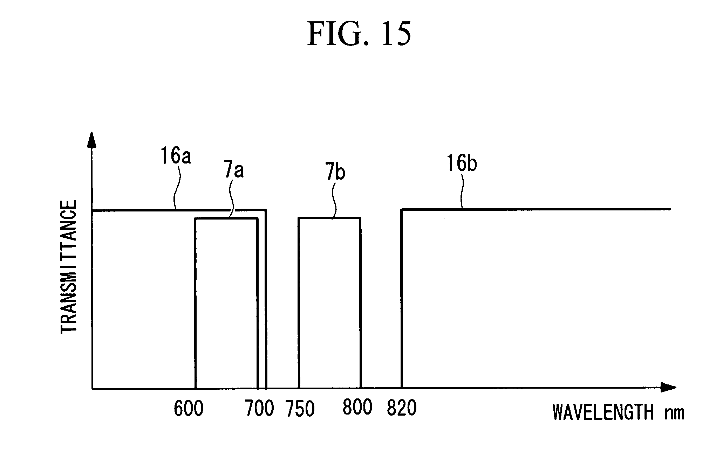

In this case, for example, as shown in Fig. 15, the rotary filter 7 at the irradiating unit 2 side may include the filter 7a for scattered light observation which transmits light in a wavelength band of 600 to 700 nm and a filter 7b for fluorescence observation which transmits light in a wavelength region of 750 to 800 nm. In addition, the rotary filter 16 at the detecting unit 3 side may include the filter 16a for scattered light observation which transmits light in a wavelength band of 700 nm or less and the filter 16b for fluorescence observation which transmits light in a wavelength region of 820 nm or more.

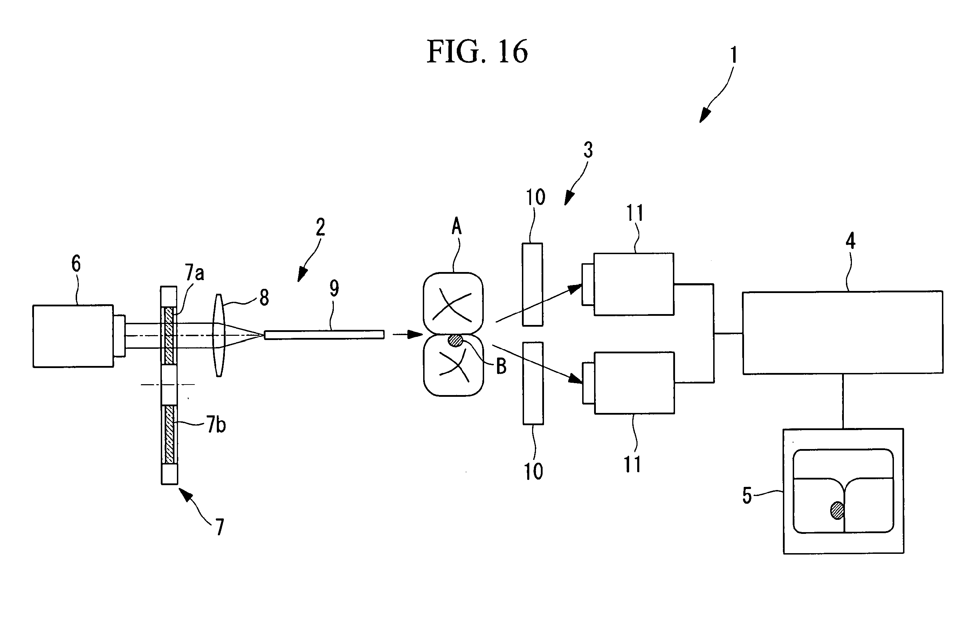

In addition, as shown in Fig. 16, two light detectors 11 may be disposed to form a vergence angle in the detecting unit 3 so as to be able to perform three-dimensional perspective observation.

With the structure described above, the spread and the degree of invasion of the caries portion B are observed in a three-dimensional manner, so that a more precise diagnosis can be performed.

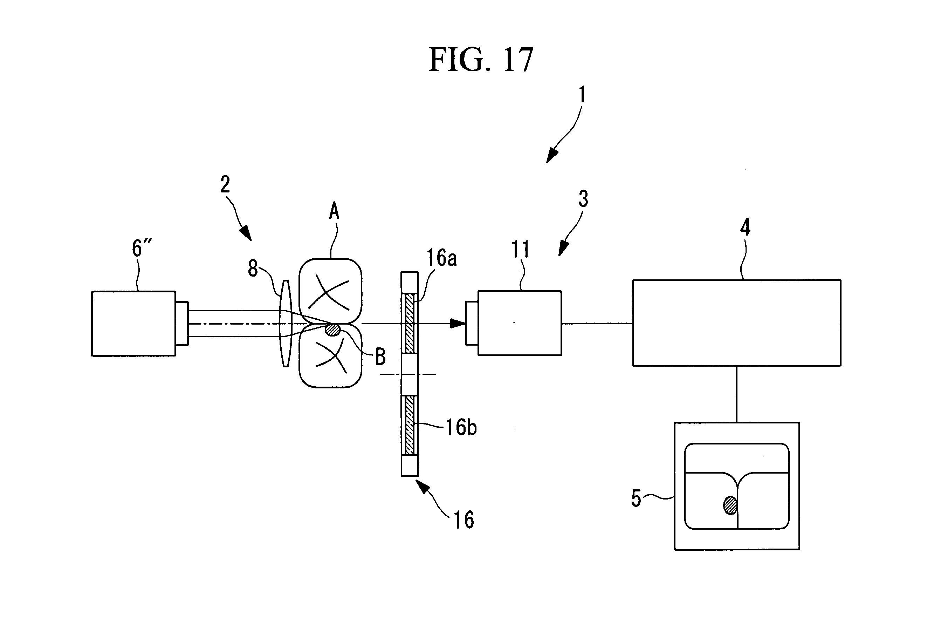

In addition, as shown in Fig. 17, as the light source, a pulsed laser light source 6" may be used, and pulsed laser light may be focused onto the caries portion B to generate second and tertiary higher harmonic waves so as to generate fluorescence.

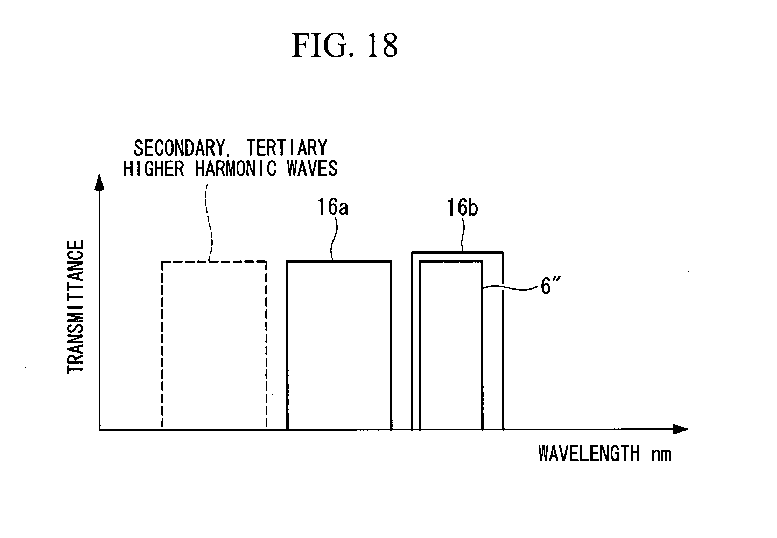

With the structure described above, as shown in Fig. 18, illumination light having a sufficiently long wavelength as compared to an excitation wavelength of a fluorescent agent can be used, thus exciting the fluorescent agent with illumination light that easily passes through the inside of the tooth A; hence, an advantage is obtained in that the caries portion B can be precisely observed.

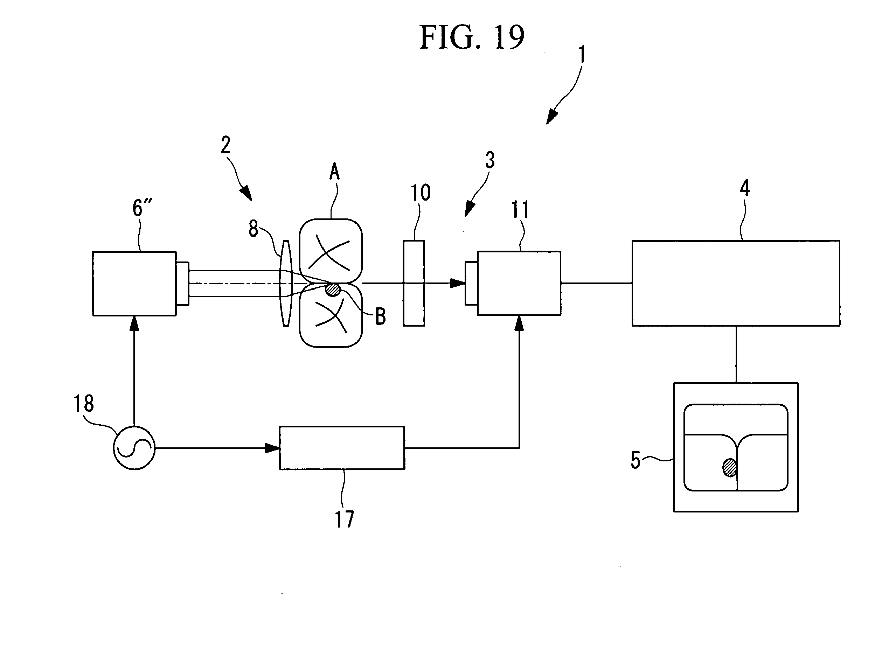

In addition, as shown in Fig. 19, the pulsed laser light source 6" may be used as the light source, and a phase adjusting device 17 which switches between a first phase for detecting the scattered light and a second phase for detecting the fluorescence may be used. Reference numeral 18 in the drawing is a frequency oscillator. Hence, using the fluorescence lifetime, the scattered light and the fluorescence can be easily separated in a time-division manner and can be observed.

In addition, in this embodiment, the image processing unit 4 may include a determination unit determining the degree of invasion (not shown).

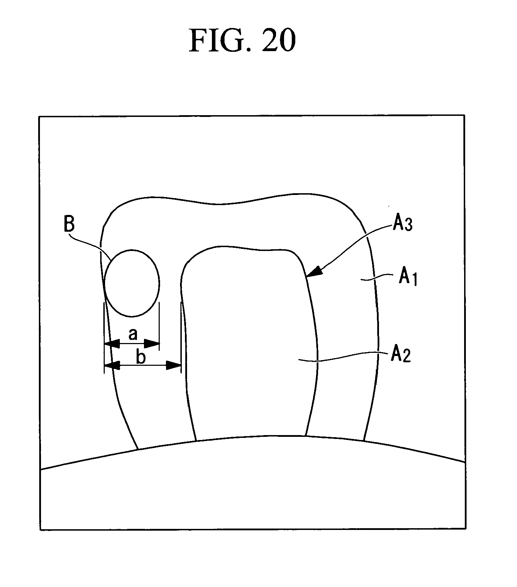

As described above, in the image processing unit 4, since the boundary A₃ between the enamel layer A₁ and the dentine layer A₂ is clearly extracted, as shown in Fig. 20, the determination unit may determine the degree of invasion of the caries portion B based on a size a of the caries portion B and a thickness dimension b of the enamel layer A₁ at a position at which the caries portion B occurs. Accordingly, when a>b holds, it may be determined that the caries portion B extends into the dentine layer A₂, and when a<b holds, it may be determined that the caries portion B remains in the enamel layer A₁. Hence, based on the determination result, a more appropriate treatment method may be advantageously selected.

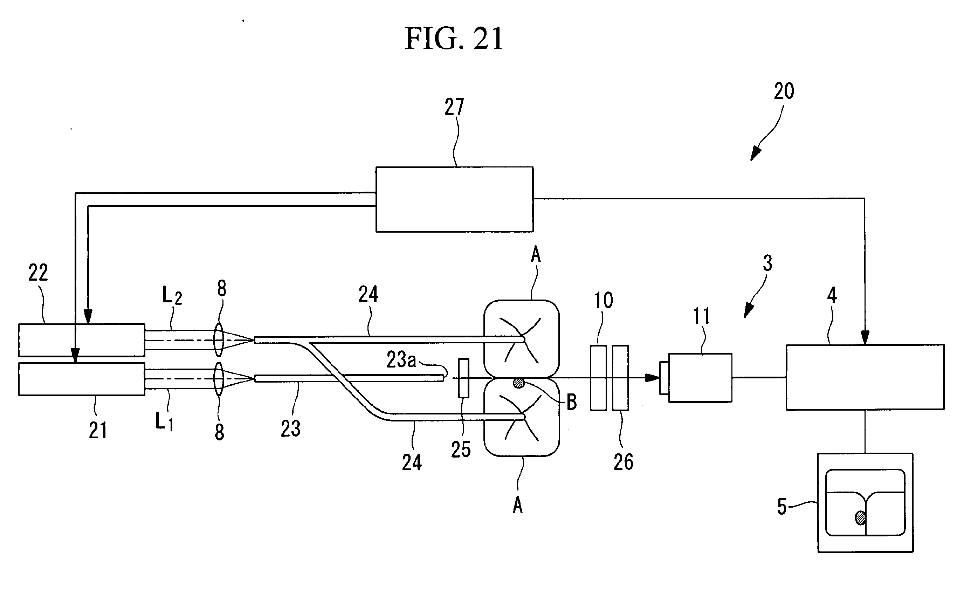

Next, a dental observation apparatus 20 according to a second embodiment of the present invention will be described with reference to Figs. 21 to 29.

In addition, in the description of this embodiment, elements and the like corresponding to those of the dental observation apparatus 1 of the above first embodiment are designated by the same reference numerals as those described above, and a description thereof is omitted.

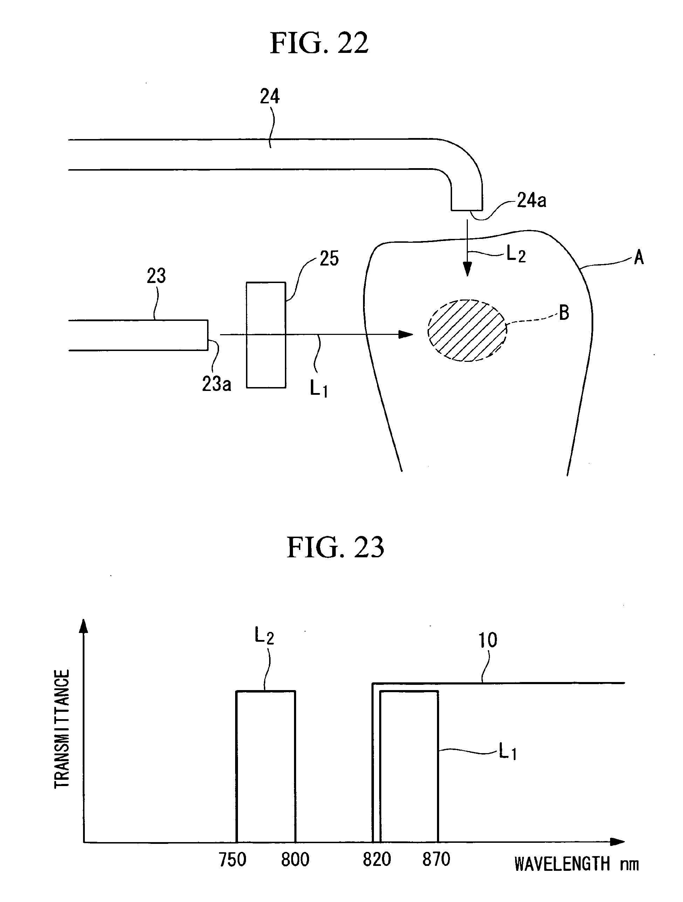

As shown in Figs. 21 and 22, the dental observation apparatus 20 of this embodiment has two light source 21 and 22 generating first illumination light L₁ and second illumination light L₂, respectively, which have different wavelength bands; optical fibers (light guiding members) 23 and 24 guiding the illumination light L₁ and the illumination light L₂ from the light sources 21 and 22, respectively; a first polarizing plate (first polarizing member) 25 disposed between the teeth A and front end surfaces (first irradiating portion and second irradiating portion) 23a and 24a of the optical fibers 23 and 24; and a second polarizing plate (second polarizing member) 26 which is disposed between the light-receiving filter 10 and a light detector 11 and which has a polarization plane different from that of the first polarizing plate 25. In addition, a control unit (illumination light switching unit) 27 is connected to the two light sources 21 and 22 and the image processing unit 4.

As shown in Fig. 23, the two light sources 21 and 22 are designed to emit light in a wavelength band of 820 to 870 nm as the first illumination light L₁ and near-infrared light in a wavelength band of 750 to 800 nm as the second illumination light L₂, respectively.

The front end surface 23a of the optical fiber 23 guiding the first illumination light L₁ is disposed in a direction so as to face a side surface in the vicinity of adjacent surfaces of the two teeth A. The optical fiber 24 guiding the second illumination light L₂ is branched into two lines, and as shown in Figs. 21 and 22, the two front surfaces 24a thereof are disposed in directions facing respective occlusion faces of the two teeth A.

The first polarizing plate 25 and the second polarizing plate 26 have different polarization planes from each other and are disposed in a so-called cross-Nicol arrangement. Accordingly, direct light or reflected light of the first illumination light L₁ emitted from the front surface 23a of the optical fiber 23 which guides the first illumination light L₁ to the side surfaces of the teeth A can be prevented from being incident on the light detector 11.

The light-receiving filter 10 is designed to block light having a wavelength of 820 nm or less.

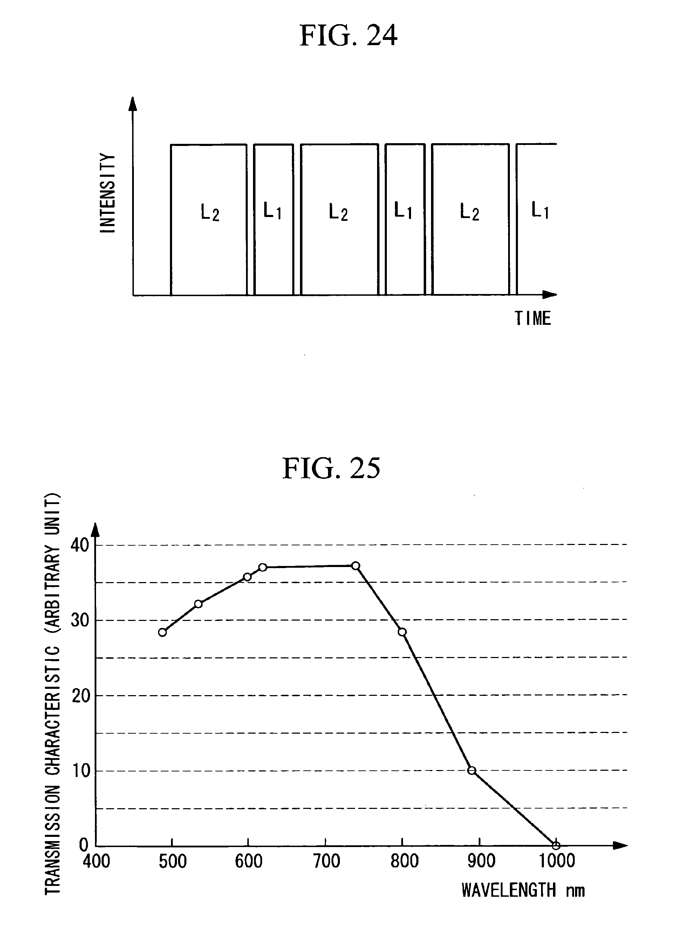

The control unit 27 is formed so that the two light sources 21 and 22 are controlled to emit the first illumination light L₁ and the second illumination light L₂ in a time-division manner, as shown in Fig. 24; the image processing unit 4 generates a scattered light image based on the intensity of scattered light detected by the light detector 11 when the first illumination light L₁ is emitted in synchronization with the switching timing of the illumination light L₁ and L₂; and the image processing unit 4 generates a fluorescence image based on the intensity of fluorescence detected by the light detector 11 when the second illumination light L₂ is emitted.

Since the intensity of the transmission light is sufficiently high when the transmission light and the fluorescence are compared with each other, in the example shown in Fig. 24, in order to ensure a sufficient exposure time for the weak fluorescence, the radiation time of the second illumination light L₂ is set to be longer than that of the first illumination light L₁.

According to the dental observation apparatus 20 of this embodiment having the structure as described above, in the scattered light image generated by the image processing unit 4 based on the intensity of the scattered light detected by the light detector 11 after the first illumination light L₁ is radiated from the side surfaces of the teeth A, the direct light and reflected light of the first illumination light L₁ are blocked by the first and the second polarizing plates 25 and 26, which are disposed in a cross-Nicol arrangement; hence, it is possible to obtain a scattered light image having a high contrast, which includes a large quantity of scattered light scattered inside the teeth A.

In addition, in the fluorescence image generated by the image processing unit 4 based on the intensity of the fluorescence which is detected by the light detector 11 after the second illumination light L₂ is radiated from the occluding surfaces of the teeth A, since the incident direction of the second illumination light L₂ and the detection direction of the fluorescence are different from each other, the second illumination light L₂ can be made difficult to detect with the light detector 11, and the incidence of the second illumination light L₂ is blocked by the light-receiving filter 10; hence, a fluorescence image having a high contrast can be obtained.

Fig. 25 shows the change in transmission characteristic of the tooth A at each light wavelength normalized by the luminance at a wavelength of 1,000 nm.

In particular, a region of interest, which is a part of the enamel in the image of the tooth A, is irradiated with light while the wavelength thereof is varied from 488 to 1,000 nm, and the average value of the luminance of the irradiated region of interest is measured. Subsequently, the average value is normalized by the exposure time, the radiation light quantity, and the radiation wavelength interval, and based on the average value of the normalized luminance at a wavelength of 1,000 nm, the transmission characteristic at each wavelength is calculated from 10×log(luminance/luminance at 1,000 nm).

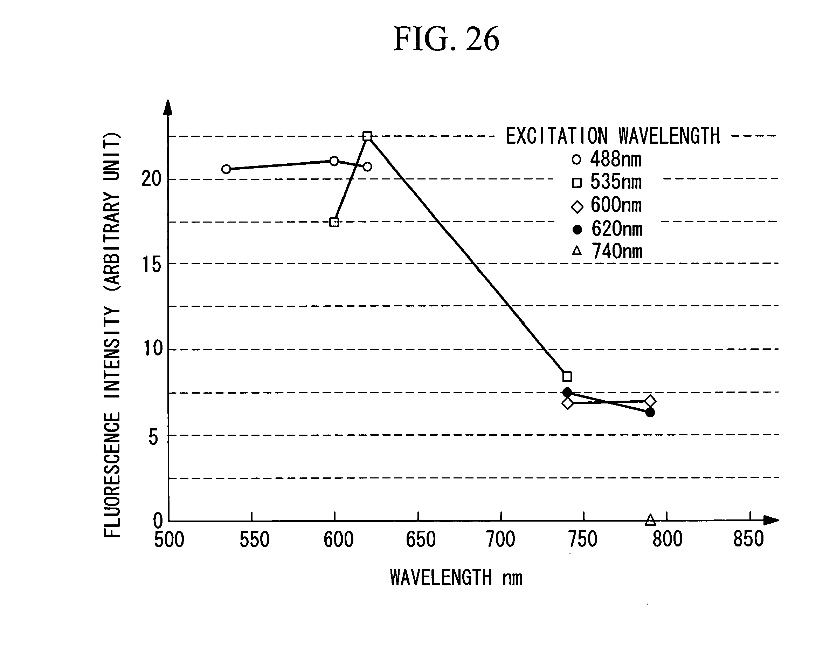

In addition, Fig. 26 shows the change in autofluorescence at each excitation wavelength, which is normalized by the luminance of autofluorescence at a wavelength of 790 nm, detected when excited by an excitation wavelength of 740 nm.

In particular, excitation light is radiated to a region of interest, which is a part of the boundary between the enamel and the dentin in an image of the tooth A, and the average value of the luminance of autofluorescence generated thereby is measured. Subsequently, the average value is normalized by the exposure time, the radiation light quantity, the radiation wavelength interval, and the detection wavelength interval, and based on the autofluorescence intensity at a wavelength of 790 nm excited by a wavelength of 740 nm, other autofluorescence intensities are calculated from 10xlog(autofluorescence intensity/autofluorescence intensity of a wavelength of 790 nm, detected when excited by an excitation wavelength of 740 nm).

According to Figs. 25 and 26, although the transmission characteristic is low in a wavelength band of 820 to 870 nm which is the first illumination light L₁ of this embodiment, since hardly any autofluorescence is generated, it is found that a scattered light image having a high contrast can be obtained. In addition, in a wavelength band of 750 to 800 nm which is the second illumination light L₂, hardly any autofluorescence is generated; hence, it is found that a fluorescence image having a high contrast can be obtained.

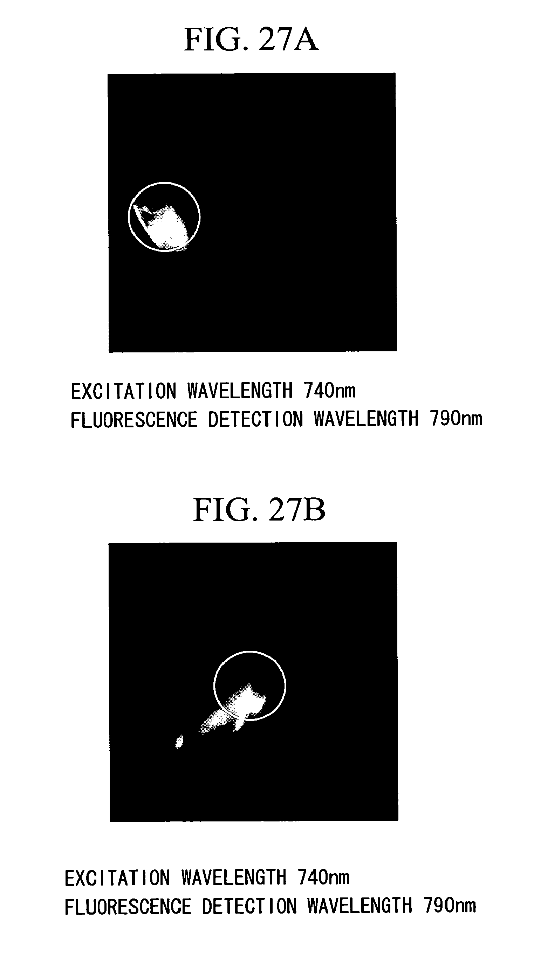

In addition, according to Figs. 27A and 27B, when the tooth A to which a fluorescent agent, namely Hilyte Fluor, is applied is irradiated with illumination light L₂ having an excitation wavelength of 740 nm, the fluorescent agent Hilyte Fluor specifically accumulated at the caries portion B is excited; hence, it is found that bright fluorescence having a wavelength 790 nm is detected on the front surface shown in Fig. 27A and the rear surface shown in Fig. 27B. Figs. 27A and 27B are fluorescence images of the caries portion B which are each obtained by radiating illumination light from a wavelength-tunable xenon light source onto a sample of a sliced molar having a thickness of 1 mm, in which the caries portion B is exposed at one surface.

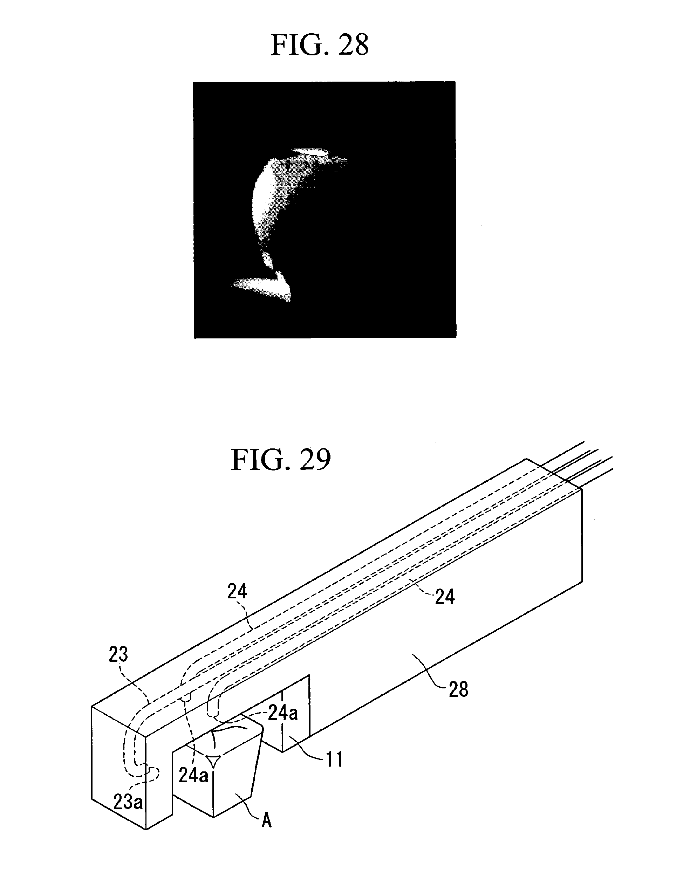

Furthermore, Fig. 28 shows a fluorescence image obtained by radiating the second illumination light L₂ from the occluding surface side. According to this image, it is found that, since direct light and reflected light of the second illumination light L₂ are not incident, a clear fluorescence image can be obtained.

In addition, since the scattered light image created by the radiation of the first illumination light L₁ and the fluorescence image created by the radiation of the second illumination light L₂ are obtained in a time-division manner, the first and the second illumination light L₁ and L₂ have no adverse influence on obtaining the fluorescence image and the scattered image, respectively, and the generation of noise in each image can be further decreased.

As shown in Fig. 29, in the dental observation apparatus 20 according to this embodiment, the front end surfaces 23a and 24a of the three optical fibers 23 and 24 are preferably disposed at a front end part of an insertion unit 28 which is to be inserted into an oral cavity, and the light detector 11 is preferably disposed at a position facing the front end surface 23a of the optical fiber 23 disposed at the frontmost side, with a space interposed therebetween. In the figure, the polarizing plates and the light-receiving filter are not shown. When the insertion unit 28 is inserted into an oral cavity, and when the front end surfaces 23a and 24a of the optical fibers 23 and 24 are extended past the teeth A and are disposed to face the rear side surfaces and the occluding surfaces, respectively, of the teeth A, the observation described above can be performed.

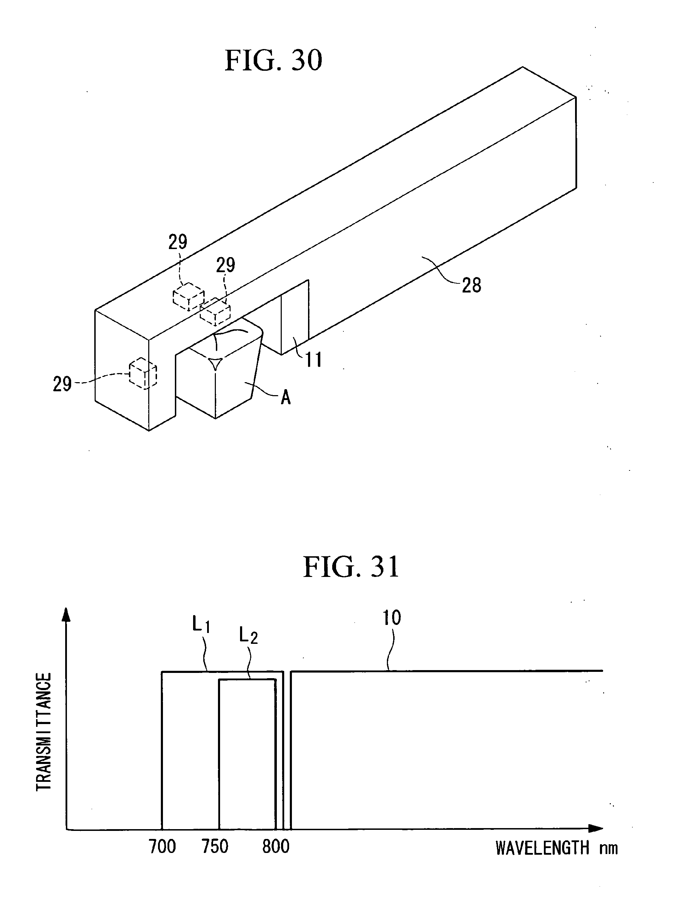

In this embodiment, as the first and the second irradiating units, the optical fibers 23 and 24 are described by way of example; however, as shown in Fig. 30, semiconductor light sources 29, such as LEDs, LDs or SLDs, may be disposed at positions of the front end surfaces 23a and 24a of the optical fibers 23 and 24. By this structure, the apparatus can be formed simply and compactly.

In addition, in this embodiment, the first illumination light L₁ and the second illumination light L₂ are different from each other; however, instead of the above structure, as shown in Fig. 31, the first illumination light L₁ and the second illumination light L₂ may have a wavelength of 700 to 800 nm and a wavelength of 750 to 800 nm, respectively, and may be overlapped with each other. In this case, as the light-receiving filter 10, a filter with a switchable transmission characteristic, such as a filter turret or a liquid crystal filter, may be used.

For example, when the first illumination light L₁ is radiated, a filter having a transmission characteristic that can transmit the first illumination light L₁ is selected as the light-receiving filter 10, and when the second illumination light L₂ is radiated, a filter that blocks the second illumination light L₂ and that has a transmission characteristic capable of transmitting fluorescence in a wavelength band of 800 to 1,000 nm is selected as the light-receiving filter 10. Accordingly, as with the case described above, a scattered light image and a fluorescence image, each having a high contrast, are obtained, and the spread and the degree of invasion of the caries portion B can be observed.

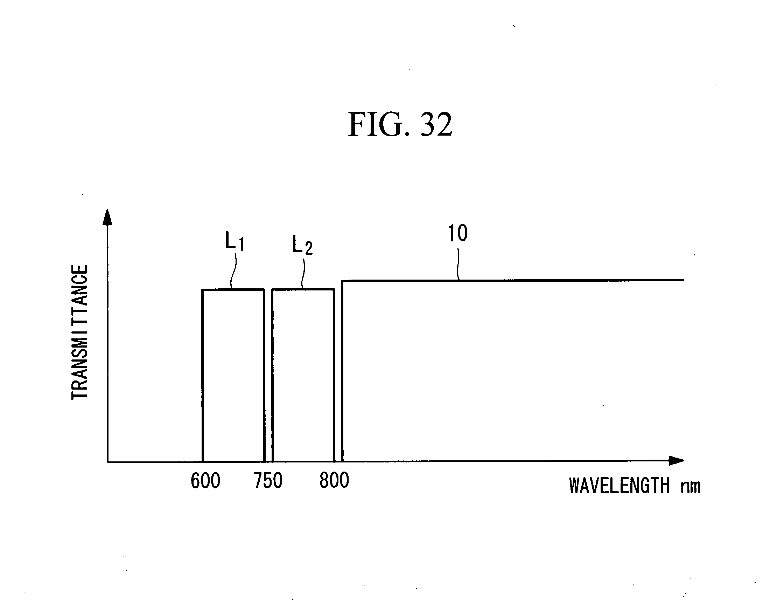

In addition, as shown in Fig. 32, the first illumination light L₁ and the second illumination light L₂ may be different from each other so as to have a wavelength of 600 to 750 nm and a wavelength of 750 to 800 nm, respectively. In this case, when the first illumination light L₁ is radiated, a filter having a transmission characteristic that can transmit the first illumination light L₁ is selected as the light-receiving filter 10, and when the second illumination light L₂ is radiated, a filter that blocks the second illumination light L₂ and that has a transmission characteristic capable of transmitting fluorescence in a wavelength band of 800 to 1,000 nm is selected as the light-receiving filter 10.

## Claims

1. A dental observation apparatus comprising:
an irradiating unit radiating illumination light including an infrared region;
a detecting unit separately detecting fluorescence generated from a caries portion of a tooth by radiation of the illumination light and scattered light of the illumination light at the tooth; and
an image processing unit which forms a fluorescence image based on the fluorescence detected by the detecting unit, which forms a scattered light image capable of identifying a boundary between an enamel layer and a dentine layer, the layers having different scattering properties, based on the intensity of the scattered light detected by the detecting unit, and which combines the fluorescence image and the scattered light image.

2. The dental observation apparatus according to Claim 1,
wherein the illumination light is near infrared light.

3. The dental observation apparatus according to Claim 1,
wherein the image processing unit compares the intensity of the scattered light obtained by the detecting unit with a predetermined threshold value and forms the scattered light image capable of identifying the boundary between the enamel layer and the dentine layer.

4. The dental observation apparatus according to Claim 1,
wherein the irradiating unit further radiates visible light,
the detecting unit further detects scattered light of the visible light, and
the image processing unit forms a scattered visible light image based on the intensity of the visible light detected by the detecting unit, so that by comparison with the scattered visible light image, the scattered light image capable of identifying the boundary between the enamel layer and the dentine layer is generated.

5. The dental observation apparatus according to Claim 1,
wherein the image processing unit performs the combining by imparting different colors to regions corresponding to the caries portion in the fluorescence image, and the enamel layer and the dentine layer in the scattered light image.

6. The dental observation apparatus according to Claim 1, further comprising:
a determination unit determining the degree of invasion of the caries portion by comparing the distance from the surface of the enamel layer to the boundary with the distance from the caries portion to the boundary.

7. The dental observation apparatus according to Claim 1,
wherein the irradiating unit includes a first irradiating unit radiating first illumination light from a side surface of the tooth and a second irradiating unit radiating second illumination light from an occluding surface of the tooth, and
the detecting unit is disposed to face the first irradiating unit with the tooth interposed therebetween.

8. The dental observation apparatus according to Claim 7, further comprising:
a first polarizing member disposed between the first irradiating unit and the tooth, and
a second polarizing member which is disposed between the tooth and the detecting unit and which has a polarization direction different from that of the first polarizing member.

9. The dental observation apparatus according to Claim 7,
wherein the second illumination light is near infrared light.

10. The dental observation apparatus according to Claim 7, further comprising:
a blocking unit which is disposed between the tooth and the detecting unit and which transmits the first illumination light and blocks the second illumination light.

11. The dental observation apparatus according to Claim 7, further comprising:
an illumination light switching unit switching between the first illumination light and the second illumination light in a time-division manner,
wherein the detecting unit detects the fluorescence or the scattered light in synchronization with switching timing of the illumination light by the illumination light switching unit.

12. The dental observation apparatus according to Claim 1, further comprising:
an insertion unit which can be inserted into an oral cavity,
wherein the irradiating unit is a semiconductor light source disposed in a front end part of the insertion unit.

13. The dental observation apparatus according to Claim 7,
wherein the first illumination light and the second illumination light are near infrared light,
further comprising an illumination light switching unit switching between the first illumination light and the second illumination light in a time-division manner, and
a blocking unit blocking the second illumination light from entering the detecting unit when the second illumination light is switched on by the illumination light switching unit,
wherein the detecting unit detects the fluorescence or the scattered light in synchronization with switching timing of the illumination light by the illumination light switching unit.
